# EUROPÄISCHE PATENTANMELDUNG

(11) **EP 2 810 694 A1**
(43) Veröffentlichungstag der Anmeldung: **10.12.2014**
(21) Anmeldenummer: 13170230.0
(22) Anmeldetag: 03.06.2013
(51) Int. Cl.: A61N 7/00, A61F 6/02, A61F 7/00

(54) **Vorrichtung und Verfahren zur Zeugungsverhütung**

(71) Anmelder: Geier, Paul, 21227 Bendestorf (DE); Grohs, Niklas, 23923 Lüdersdorf (DE)
(72) Erfinder: Geier, Paul, 21227 Bendestorf (DE); Grohs, Niklas, 23923 Lüdersdorf (DE)

(57) **Zusammenfassung**

Die Erfindung betrifft ein Verfahren und eine Vorrichtung zur Zeugungsverhütung bei männlichen Säugern.

Derartige Vorrichtungen sind aus dem Stand der Technik auf zahlreiche Art und Weise bekannt. Aufgabe der vorliegenden Erfindung ist es, derartige Vorrichtungen und Verfahren zu verbessern und insbesondere für die Anwendung im Alltag praktikabler und zuverlässiger auszugestalten.

Gelöst wird die Aufgabe durch ein Verfahren zur Zeugungsverhütung bei männlichen Säugern, insbesondere bei Männern, wobei die Hoden des Säugers durch Wärmeleitung und/oder Strahlung erwärmt und währenddessen mit Ultraschall bestrahlt werden. Dabei weist der Ultraschall eine Frequenz zwischen 1,5 und 4,5 MHz auf. Der kombinierte Effekt der Erwärmung durch Wärmeleitung und Strahlung und die durch den Ultraschall bewirkte Erwärmung sorgt für eine Zerstörung von Proteinen und dadurch für eine zuverlässige Reduzierung der Fertilität der Spermien.

## Beschreibung

Die Erfindung betrifft ein Verfahren und eine Vorrichtung zur Zeugungsverhütung bei männlichen Säugern.

Derartige Vorrichtungen sind aus dem Stand der Technik auf zahlreiche Art und Weise bekannt.

So sind besonders bekannt Kondome. Darüber hinaus ist es auch bekannt, die Zeugungsfähigkeit durch Wärmeeinwirkung zu reduzieren bzw. durch Wärmeeinwirkung eine Zeugungsverhütung zu erreichen. Dies ist beispielsweise bekannt aus der DE 299 10 215 oder der DE 100 27 378.

Darüber hinaus ist es auch bekannt, die Zeugungsfähigkeit durch Einwirkung von Ultraschall zu verringern. Dies geht beispielsweise aus der DE 27 15 855 oder der US 2009/0171138 hervor.

Aufgabe der vorliegenden Erfindung ist es, derartige Vorrichtungen und Verfahren zu verbessern und insbesondere zur Verwendung im Alltag praktikabler und zuverlässiger auszugestalten.

Gelöst wird die Aufgabe durch ein Verfahren gemäß Anspruch 1 sowie eine Vorrichtung gemäß Anspruch 9. Die abhängigen Ansprüche 2 bis 8 sowie 10 bis 15 geben vorteilhafte Weiterbildungen an.

Erfindungsgemäß zeichnet sich das Verfahren zur Zeugungsverhütung bei männlichen Säugern, insbesondere bei Männern, dadurch aus, dass die Hoden des Säugers durch Wärmeleitung und/oder Strahlung erwärmt und währenddessen mit Ultraschall bestrahlt werden. Dabei weist der Ultraschall eine Frequenz zwischen 1,5 und 4,5 MHz auf. Der kombinierte Effekt der Erwärmung durch Wärmeleitung und/oder -strahlung die durch den Ultraschall bewirkte Erwärmung sorgt für eine Zerstörung von Proteinen und dadurch für eine zuverlässige Reduzierung der Fertilität der Spermien.

Somit lassen sich durch dieses Verfahren deutlich zuverlässige Zeugungsverhütungsergebnisse erzielen, als dies bei üblichen Verfahren, wie beispielsweise Kondomen, der Fall ist.

Darüber hinaus zeichnet sich das Verfahren dadurch aus, dass es nicht während des Geschlechtsverkehrs zum Einsatz kommen muss, um seine Wirkung zu zeigen.

Die erfindungsgemäße Erwärmung durch Wärmeleitung und/oder Strahlung kann dabei auf unterschiedlichste Weise erzielt werden. Üblicherweise erfolgt die Erwärmung der Hoden dabei indirekt durch Erwärmung des Hodensacks. Dies kann beispielsweise durch entsprechende Heizleiter oder anderes bewirkt werden, die entsprechend am Hodensack und/oder in thermischen Kontakt mit diesem platziert werden.

Entscheidend kommt es auch auf die Wahl der Frequenz zwischen 1,5 und 4,5 MHz an. Niedrigere Frequenzen führen bei erforderlichen Energien zu Schädigungen am Gewebe, während Strahlung höherer Frequenzen als 4,5 MHz nicht ausreichend bis zu den Hoden bzw. Nebenhoden vordringen kann, wenn sie von außen appliziert wird.

Eine solche äußere Applizierung der Ultraschallwellen ist Gegenstand dieser Erfindung. Das Verfahren ist somit nicht invasiv. Zur Bestrahlung mit Ultraschall wird dementsprechend excorporal ein Ultraschallerzeuger platziert. Er wird insbesondere durch Ultraschallkoppelmittel mit dem Hodensack gekoppelt.

Mit besonderem Vorteil wird die Frequenz zur Steigerung der Effizienz darüber hinaus zwischen 1,5 und unterhalb von 4 MHz gewählt. Dabei bevorzugt wird eine Frequenz zwischen 2,5 und 3,5 MHz.

Zur Durchführung des Verfahrens wird insbesondere der Hodensack in Ultraschallkoppelmittel getaucht und das Ultraschallkoppelmittel durch entsprechende Heizvorrichtungen erwärmt.

Mit besonderem Vorteil wird die Ultraschallbestrahlung gemäß Anspruch 2 gepulst durchgeführt. Eine solche gepulste Bestrahlung kann in unterschiedlichsten Bestrahlungszyklen realisiert werden. Bevorzugt ist es jedoch, wenn derartige Zyklen Erholungsphasen ohne Ultraschallbestrahlung beinhalten, die eine Dauer von mehreren Sekunden aufweisen. Dadurch lässt sich eine besonders effiziente Zeugungsverhütung realisieren, ohne Schädigungen in Kauf zu nehmen.

Dabei wird es insbesondere bevorzugt, wenn in jedem Intervall von 160 Sekunden, in dem mit Ultraschall bestrahlt wird, maximal 90 Sek. lang mit Ultraschall bestrahlt wird und mindestens 30 Sek. lang keine Bestrahlung mit Ultraschall durchgeführt wird. Dabei müssen die einzelnen Phasen von Bestrahlung und Ruhe nicht zusammenhängend gewählt werden. So kann beispielsweise eine Abfolge von 20 Sekunden Bestrahlung und 10 Sekunden Ruhe gewählt werden.

Mit besonderem Vorteil wird eine maximale Ultraschallleistung von 20 Watt, insbesondere maximal 15 Watt eingesetzt, wobei die Bestrahlungsleistung pro Minute vorteilhafterweise 2 bis 15 Watt beträgt und insbesondere zwischen 4 und 12 Watt pro Minute liegt. Besonders bevorzugt wird eine Bestrahlung pro Behandlung mit 30 bis 180 Watt. Vorteilhafterweise beträgt die Leistung mindestens 2 Watt.

Mit Vorteil wird die Erwärmung der Hoden durch Wärmeleitung und/oder Wärmestrahlung so gewählt, dass allein durch sie eine Erwärmung des Hodensacks und/oder der Hoden auf mehr als 40°C erfolgt bzw. erfolgen würde, falls kein Ultraschall zeitgleich bzw. währenddessen eingesetzt wird. Besonders bevorzugt wird eine Erwärmung auf über 42°C durch die Wärmeleitung und/oder Wärmestrahlung. Noch bessere Ergebnisse oder kürzere Anwendungsdauern können erzielt werden, wenn eine Erwärmung allein durch Wärmeleitung und/oder - strahlung auf mindestens 45°C erfolgt. Es hat sich jedoch herausgestellt, dass höhere Temperaturen häufig nicht schmerzfrei toleriert werden können. Insbesondere eine Erwärmung allein durch Wärmeleitung und/oder -strahlung auf nicht mehr als 50°C durchgeführt.

Mit besonderem Vorteil wird die Erwärmung durch Wärmeleitung und/oder -strahlung durch Heizen mit einem Heizleiter bewirkt. Bei einem solchen Heizleiter kann es sich beispielsweise um einen Heizdraht handeln.

Mit besonderem Vorteil wird das Verfahren so durchgeführt, dass die Erwärmung und Bestrahlung der Hoden des männlichen Säugers ein- bis zweimal pro 24 bis 48 Stundenan mindestens vier, insbesondere zwei, aufeinanderfolgenden Tagen erfolgt, so dass also ein bis zwei Behandlungen pro 24 bis 28 Stundenan mindestens vier, insbesondere zwei, aufeinanderfolgenden Tagen erfolgen. Die Behandlungen dauern dabei vorteilhafterweise fünf bis 30 Minuten. Diese fünf bis 30 Minuten sind die Dauer des Verfahrens, während dessen eine Erwärmung der Hoden durch Wärmeleitung und/oder -strahlung stattfindet. Während dieser Zeit wird auch die Bestrahlung mit Ultraschall durchgeführt. Sie muss jedoch nicht über die ganze Zeitspanne hinweg andauern. Auch bei Verwendung von entsprechenden Zyklen oder gepulster Ultraschallstrahlung können diese Zyklen auch nur in Teilbereichen dieser Zeitspanne angewandt werden. Durch eine solche Anwendung ein- bis zweimal pro 24 bis 48 Stundenan mindestens vier, insbesondere zwei, aufeinanderfolgenden Tagen von je fünf bis 30 Minuten kann die Fertilität für etwa zwei bis drei Monate ausreichend reduziert werden.

Es ist beispielsweise vorteilhaft, zunächst eine Erwärmung der Hoden auf die gewünschte Temperatur allein durch Wärmestrahlung bzw. Wärmeleitung zu bewirken und erst bei Erreichen einer bestimmten Temperatur durch diese Erwärmung mit der Ultraschallbestrahlung zu beginnen.

Mit besonderem Vorteil kann die Temperatur, auf die die Hoden und/oder der Hodensack und/oder das Ultraschallkoppelmittel erwärmt werden/wird, insbesondere allein durch Wärmeleitung und/oder -strahlung vom Anwender in einer vorgegebenen Spanne, insbesondere zwischen 42°C und 50°C verändert werden und wird die Dauer und/der Intensität der Bestrahlung an die gewählte Temperatur angepasst, insbesondere der Art, das die Dauer und/oder Intensität bei höheren Temperaturen kürzer gewählt wird als bei niedrigeren Temperaturen.

So kann beispielsweise bei einer Temperatur des Ultraschallkoppelmittels von 42°C eine Ultraschallbestrahlungsdauer von 10 bis 15 min ausreichen, während bei einer Temperatur von 48°C schon eine Bestrahlungsdauer von 2 bis 7 Minuten bei gleicher Intensität ausreichen kann.

Mit besonderem Vorteil werden die Hoden des Säugers zur Bestrahlung in ein Behältnis mit Ultraschallkoppelmittel aufgenommen. In das Ultraschallkoppelmittel, das sich im Behältnis befindet, werden die Ultraschallwellen eingekoppelt.

Eine solche Anordnung ist besonders flexibel auf unterschiedliche anatomische Formen anwendbar und effizient. Vorteilhafterweise wird in einem solchen Fall die Erwärmung durch Wärmeleitung und/oder -strahlung durch Erwärmung des Ultraschallkoppelmittels durchgeführt. Dies kann beispielsweise durch entsprechende Heizelemente zur Heizung des Ultraschallkoppelmittels erfolgen. So ist es auch denkbar, vor Aufnahme der Hoden in das Behältnis mit Ultraschallkoppelmittel, dieses Behältnis und das Ultraschallkoppelmittel entsprechend vorzuheizen. Dabei kann die Heizung auf eine angenehme Temperatur, zwischen 33 und 37°C erfolgen oder auch schon eine entsprechende Vorheizung in Richtung der gewünschten Zieltemperatur, also beispielsweise auf eine Temperatur zwischen 40 und 45°C erfolgen. Letzteres verkürzt die notwendige Behandlungsdauer weiter.

Mit besonderem Vorteil wird die Temperatur bzw. die Wärmeeinbringung durch Wärmeleitung und Wärmestrahlung, beispielsweise die Temperatur des Ultraschallkoppelmittels oder des umgebenden Gefäßes durch einen entsprechenden Sensor erfasst und überwacht und insbesondere die Erwärmung durch Wärmestrahlung und Wärmeleitung basierend auf dieser Erfassung geregelt. So kann beispielsweise die Speisung eines Heizdrahtes entsprechend geregelt werden. Mit besonderem Vorteil wird auch die Erzeugung bzw. die Einkopplung der Ultraschallwellen überwacht. So kann beispielsweise ein entsprechender Ultraschallsensor verwendet werden. Bei Verwendung von Ultraschallkoppelmittel zur Einkopplung wird vorteilhafterweise dieser Sensor ebenfalls mit dem Ultraschallkoppelmittel in Verbindung gebracht. Dadurch kann sichergestellt werden, dass die beabsichtigte Ultraschallbestrahlung auch durchgeführt wird und nicht ein Defekt der Ultraschallerzeugung unerkannt bleibt.

Die Aufgabe wird auch gelöst durch eine Vorrichtung zur Zeugungsverhütung bei männlichen Säugern, aufweisend einen Ultraschallerzeuger zur Erzeugung von Ultraschall mit einer Frequenz zwischen 1,5 und 4,5 MHz und einer Heizvorrichtung zur Erwärmung der Hoden des Säugers durch Wärmeleitung und/oder -strahlung. Dabei ist die Vorrichtung so eingerichtet, dass die Hoden des Säugers während der Erwärmung der Hoden des Säugers durch die Heizvorrichtung durch den Ultraschallerzeuger mit Ultraschall bestrahlt werden. Eine solche Ultraschallbestrahlung muss nicht über die gesamte Zeitdauer der Erwärmung durch die Heizvorrichtung andauern. Sie muss nur während der Erwärmung der Heizvorrichtung erfolgen.

Entscheidend kommt es auch auf die Wahl der Frequenz zwischen 1,5 und 4,5 MHz an. Niedrigere Frequenzen führen bei erforderlichen Energien zu Schädigungen am Gewebe, während Ultraschall höherer Frequenzen nicht ausreichend bis zu den Hoden bzw. Nebenhoden vordringen kann, wenn er von außen appliziert wird. Für eine solche äußere Applizierung der Ultraschallwellen ist die Vorrichtung eingerichtet. Die Vorrichtung ist somit nicht zur Verbringung in den Körper oder in Körperöffnungen eingerichtet. Zur Bestrahlung mit Ultraschall wird dementsprechend ein excorporalern Ultraschallerzeuger der erfindungsgemäßen Vorrichtung excorporal platziert. Somit weist die erfindungsgemäße Vorrichtung eine entsprechende Einkopplungseinrichtung oder Fläche zur excorporalen Einkopplung der Ultraschallwellen des Ultraschallerzeugers auf. Der Ultraschall wird insbesondere durch Ultraschallkoppelmittel mit dem Hodensack gekoppelt. Dazu weist die Vorrichtung insbesondere eine entsprechende Koppelfläche zur Benetzung und/oder eine Aufnahme von Ultraschallkoppelmittel zur Einkopplung der Ultraschallwellen in den Körper, insbesondere den Hodensack, des Säugers auf.

Mit besonderer Effizienz wird die Frequenz darüber hinaus zwischen 1,5 und unterhalb von 4 MHz gewählt. Dabei für besondere Effizienz bevorzugt wird eine Frequenz zwischen 2,5 und 3,5 MHz.

Bezüglich der einzelnen Ausgestaltungen, Weiterentwicklungen und Vorteile gilt oben zum Verfahren Ausgeführtes.

Mit besonderem Vorteil weist die Vorrichtung eine Steuerungseinheit zur Steuerung der Erwärmung der Hoden des Säugers durch Wärmeleitung und/oder -strahlung und der Ultraschallbestrahlung auf. Die Steuerungseinheit kann zur Steuerung der Erwärmung der Hoden des Säugers durch Wärmeleitung und/oder -strahlung beispielsweise eine Steuerung eines entsprechenden Heizdrahtes bzw. einer entsprechenden Heizvorrichtung umfassen. Auch kann es eingerichtet sein, die Frequenz zwischen 1,5 und 4,5 MHz zu steuern. Die Steuerungseinheit kann dabei von der Heizvorrichtung und dem Ultraschallerzeuger räumlich getrennt oder in direktem Verbund mit diesen angeordnet sein. Bei räumlicher Trennung ist für eine entsprechende Kopplung, beispielsweise durch entsprechende Leitungen, zu sorgen. Die Steuerungseinheit kann dabei Bedienelemente und Anzeigeeinheiten sowie auch Energieversorgungsmittel, beispielsweise Netzteil und/oder Batterien bzw. Akkus enthalten.

Die Steuerungseinheit ist dabei insbesondere dazu eingerichtet, die Bestrahlung mit Ultraschall während der Erwärmung durch die Heizvorrichtung durchzuführen. Sie kann auch eingerichtet sein, die bezüglich des erfindungsgemäßen Verfahrens geschilderten Verfahrensschritte einzeln oder in einer beliebigen Kombination durchzuführen bzw. die entsprechenden Mittel entsprechend zu steuern.

Insbesondere ist die Vorrichtung und/oder Steuerungseinheit gemäß Anspruch 11 zur gepulsten Bestrahlung mit Ultraschall eingerichtet. Bezüglich der Art der gepulsten Bestrahlung gilt ebenfalls oben Ausgeführtes.

Mit besonderem Vorteil weist die Vorrichtung mindestens ein Eingabemittel zur Vorwahl der Heizleistung der Heizvorrichtung und/oder zur Vorwahl der Erwärmung durch Wärmeleitung und/oder -strahlung und/oder der Temperatur, auf die der Hodensack und/oder die Hoden und/oder das Ultraschallkoppelmittel, insbesondere durch die Heizvorrichtung, erwärmt werden/wird. Insbesondere ist die Steuerungseinheit eingerichtet, eine gemessene Temperatur des Hodensacks und/oder des Ultraschallkoppelmittel und/oder die Vorwahl der Heizleistung der Heizvorrichtung und/oder der Vorwahl der Erwärmung durch Wärmeleitung und/oder -strahlung und/oder der Temperatur, auf die der Hodensack und/oder die Hoden und/oder das Ultraschallkoppelmittel allein durch Wärmeleitung und/oder -strahlung erwärmt werden/wird, bei der Ansteuerung des Ultraschallerzeugers, insbesondere hinsichtlich der Intensität und/oder Dauer der Ultraschallerzeugung, zu berücksichtigen, insbesondere dahin gehend, dass die Dauer und/oder Intensität der Ultraschallerzeugung bei höheren Temperaturen und/oder Heizleistungen geringer gewählt wird, als bei niedrigeren Temperaturen und/oder Heizleistungen.

Mit besonderem Vorteil weist die Vorrichtung gemäß Anspruch 12 ein Behältnis zur Aufnahme von Ultraschallkoppelmittel und der Hoden des Säugers auf. Dabei ist die Heizvorrichtung so angeordnet, dass sie geeignet ist, das Ultraschallkoppelmittel zu erwärmen. Der Ultraschallerzeuger ist so angeordnet, dass er geeignet ist, Ultraschall in das Ultraschallkoppelmittel einzukoppeln. Eine solche Vorrichtung ist besonders praktisch und effizient einsetzbar sowie kostengünstig und platzsparend herstellbar.

Mit besonderem Vorteil ist das Behältnis gemäß Anspruch 13 aus Ultraschall reflektierendem, insbesondere elektrisch leitendem, Material , insbesondere Metall besteht und/oder zumindest seine zum Ultraschallkoppelmittel gerichtete Wandung aus einem solchen Material ausgebildet oder damit beschichtet ist. Mit weiterem Vorteil ist das Behältnis thermisch isoliert. Mit besonderem Vorteil kann hier ein doppelwandiges Behältnis mit Gas bzw. insbesondere Luft gefülltem Zwischenraum verwendet werden. Vorteilhafterweise werden in diesem Zwischenraum die entsprechenden Elemente, wie Heizvorrichtung, Sensoren und/oder Ultraschallerzeuger angeordnet. Als Sensoren werden vorteilhafterweise ein Temperatursensor und/oder ein Ultraschallsensor eingesetzt.

Mit weiterem Vorteil weist das Behältnis an einer Unterseite, insbesondere einer Unterseite, die einer Öffnung zur Einführung der Hoden gegenüberliegt, den Ultraschallerzeuger auf. Mit Vorteil ist die Heizvorrichtung an den Seiten des Behältnisses angeordnet. Insbesondere ist sie das Behältnis an diesen Seiten umschließend angeordnet. So kann beispielsweise ein Heizdraht um die innere Wandung des Behältnisses gewickelt sein.

Mit weiterem Vorteil ist die Heizvorrichtung so gewählt und/oder die Steuerungseinheit so eingerichtet, dass allein durch die Heizvorrichtung eine Erwärmung des Hodensacks und/oder der Hoden auf mehr als 40°C, insbesondere auf 42°C oder mehr, mit weiterem Vorteil auf 45°C oder mehr, insbesondere maximal 50°C erfolgt.

Mit weiterem Vorteil weist das Behältnis eine Verschlussvorrichtung zur sicheren Haltung des Hodensacks bzw. der Hoden im Behältnis und insbesondere in dem Ultraschallkoppelmittel auf.

Mit besonderem Vorteil weist die Vorrichtung Mittel zum Loggen der Daten der Behandlung, insbesondere der Erwärmung und Bestrahlung und/oder der Sensordaten auf.

Mit besonderem Vorteil ist die Steuerungseinheit so eingerichtet, dass ein zeitlicher Mindestabstand zwischen den einzelnen Behandlungen bzw. Durchführungen der Erwärmungen und Bestrahlungen eingehalten werden muss und während dieses Zwischenraumes eine weitere Behandlung oder Durchführung des Verfahrens bzw. Erwärmung oder Bestrahlung nicht möglich ist. Dieses Intervall beträgt vorteilhafterweise 3 bis 48, insbesondere 6 bis 36 Stunden.

Weitere vorteilhafte Ausbildungen und Anpassungen sollen im Folgenden rein exemplarisch und nicht beschränkend anhand der folgenden schematischen Zeichnung erläutert werden. Dabei zeigt die Figur:

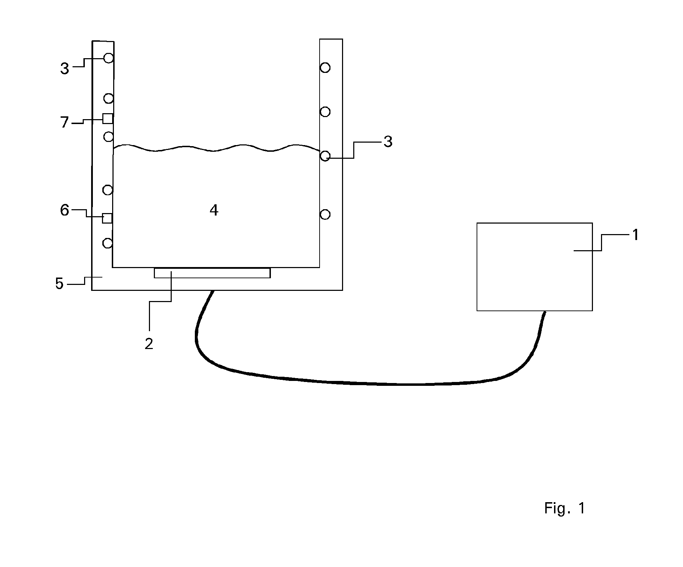

- Fig. 1: eine schematische Ansicht einer erfindungsgemäßen Vorrichtung.

Fig. 1 zeigte eine erfindungsgemäße Vorrichtung. Zu erkennen ist eine Steuerungseinheit 1, die über eine Leitung mit einem Behältnis 5 und den darin angeordneten Elementen verbunden ist. Das Behältnis 5 ist doppelwandig ausgeführt. In dem zwischen den Wandungen sich ergebenden Zwischenraum ist ein Ultraschallerzeuger 2 angeordnet. Darüber hinaus sind dort angeordnet ein um die Seitenwand gewickelter Heizdraht 3 sowie ein Temperatursensor 7 und ein Ultraschallsensor 6. Das Behältnis ist in seinem Innenraum hälftig mit Ultraschallkoppelmittel 4 gefüllt. In das Behältnis 5 kann durch seine Öffnung ein Hodensack eingeführt werden. Dieser verdrängt dann Ultraschallkoppelmittel 4, so dass ein höherer Füllstand des Ultraschallkoppelmittels 4 im Behälter 5 zustande kommt. Durch entsprechende Eingaben bzw. Bedienungen an der Steuerungseinheit 1 werden dann die Heizvorrichtung 3 und der Ultraschallerzeuger 2 angesteuert sowie die Messdaten des Temperatursensors 7 und des Ultraschallsensors 6 überwacht und zur Regelung verarbeitet.

**Bezugszeichenliste:**

| | |
|---|---|
| 1 | Steuerungseinheit |
| 2 | Ultraschallerzeuger |
| 3 | Heizdraht |
| 4 | Ultraschallkoppelmittel |
| 5 | Thermobecher |
| 6 | Temperatursensor |
| 7 | Ultraschallsensor |

## Patentansprüche

1. Verfahren zur Zeugungsverhütung bei männlichen Säugern, wobei die Hoden des Säugers durch Wärmeleitung und/oder -strahlung erwärmt und währenddessen mit Ultraschall bestrahlt werden, wobei der Ultraschall eine Frequenz zwischen 1,5 und 4,5 MHz aufweist.

2. Verfahren nach dem vorstehenden Anspruch, wobei die Bestrahlung mit Ultraschall gepulst durchgeführt wird.

3. Verfahren nach einem der vorstehenden Ansprüche, wobei in jedem Intervall von 160 Sekunden, in dem mit Ultraschall bestrahlt wird, maximal 90 Sekunden lang mit Ultraschall bestrahlt wird und mindestens 30 Sekunden lang keine Bestrahlung mit Ultraschall durchgeführt wird.

4. Verfahren nach einem der vorstehenden Ansprüche, wobei die Erwärmung der Hoden durch Wärmeleitung und/oder -strahlung so gewählt wird, dass allein durch sie eine Erwärmung des Hodensacks und/oder der Hoden auf mehr als 40°C erfolgt.

5. Verfahren nach einem der vorstehenden Ansprüche, wobei die Erwärmung durch Wärmeleitung und/oder -strahlung durch Heizen mit einem Heizleiter bewirkt wird.

6. Verfahren nach einem der vorstehenden Ansprüche, wobei die Erwärmung und Bestrahlung der Hoden des Säugers ein- bis zweimal pro 24 bis 48 Stunden an mindestens vier aufeinanderfolgenden Tagen erfolgt.

7. Verfahren nach einem der vorstehenden Ansprüche, wobei die Hoden des Säugers zur Bestrahlung in ein Behältnis mit Ultraschallkoppelmittel aufgenommen werden.

8. Verfahren nach dem vorstehenden Anspruch, wobei die Erwärmung durch Wärmeleitung und/oder -strahlung durch Erwärmung des Ultraschallkoppelmittels erfolgt.

9. Vorrichtung zur Zeugungsverhütung bei männlichen Säugern, aufweisend einen Ultraschallerzeuger (2) zur Erzeugung von Ultraschall mit einer Frequenz zwischen 1,5 und 4,5 MHz und eine Heizvorrichtung (3) zur Erwärmung der Hoden des Säugers durch Wärmeleitung und/oder -strahlung, wobei die Vorrichtung eingerichtet ist, die Hoden des Säugers während der Erwärmung der Hoden des Säugers durch die Heizvorrichtung (3) durch den Ultraschallerzeuger (2) mit Ultraschall zu Bestrahlen.

10. Vorrichtung nach dem vorstehenden Anspruch 9 aufweisend eine Steuerungseinheit (1) zur Steuerung der Erwärmung der Hoden des Säugers durch Wärmeleitung und/oder -strahlung und der Ultraschallbestrahlung.

11. Vorrichtung nach einem der vorstehenden Anspruch 9 bis 10, wobei die Vorrichtung und/oder Steuerungseinheit (1) zur gepulsten Bestrahlung mit Ultraschall eingerichtet ist.

12. Vorrichtung nach einem der vorstehenden Ansprüche 9 bis 11 aufweisend ein Behältnis (5) zur Aufnahme von Ultraschallkoppelmittel (4) und der Hoden des Säugers, wobei in die Heizvorrichtung (3) so angeordnet ist, dass sie geeignet ist, das Ultraschallkoppelmittel (4) zu erwärmen und wobei der Ultraschallerzeuger (2) angeordnet ist, Ultraschall in das Ultraschallkoppelmittel (4) einzukoppeln.

13. Vorrichtung nach dem vorstehenden Anspruch 12, wobei das Behältnis (5) aus Ultraschall reflektierendem Material besteht oder zumindest seine zum Ultraschallkoppelmittel (4) gerichtete Wandung aus einem solchen Material ausgebildet oder damit beschichtet ist.

14. Vorrichtung nach einem der vorstehenden Ansprüche 12 bis 13, wobei das Behältnis (5) an einer Unterseite den Ultraschallerzeuger (2) aufweist und die Heizvorrichtung (3) an den Seiten des Behältnisses angeordnet ist.

15. Vorrichtung nach einem der vorstehenden Ansprüche 9 bis 14, wobei die Heizvorrichtung (3) so gewählt und/oder die Steuerungseinheit (1) so eingerichtet ist/sind, dass allein durch die Heizvorrichtung (3) eine Erwärmung des Hodensacks und/oder der Hoden auf mehr als 40 °C erfolgt.
